(19) Europäisches Patentamt
European Patent Office
Office européen des brevets

(11) Veröffentlichungsnummer: **0 195 895 B1**

(12) # EUROPÄISCHE PATENTSCHRIFT

(45) Veröffentlichungstag der Patentschrift: **19.08.92**

(51) Int. Cl.$^5$: **A61K 7/08**

(21) Anmeldenummer: **86100998.3**

(22) Anmeldetag: **25.01.86**

(54) **Mittel zum Waschen oder Spülen der Haare.**

(30) Priorität: **02.02.85 DE 3503618**

(43) Veröffentlichungstag der Anmeldung:
**01.10.86 Patentblatt 86/40**

(45) Bekanntmachung des Hinweises auf die Patenterteilung:
**19.08.92 Patentblatt 92/34**

(84) Benannte Vertragsstaaten:
**AT BE CH DE FR GB IT LI NL SE**

(56) Entgegenhaltungen:
**EP-A- 0 056 595**
**EP-A- 0 116 439**
**EP-A- 0 188 216**
**JP-A-54 129 135**

**CHEMICAL ABSTRACTS, Band 88, Nr. 22, Mai 1978, Columbus, OH (US); Nr. 158285y**

**THE JOURNAL OF THE AMERICAN OIL CHEMISTS SOCIETY, Band 52, Nr. 7, The American Oil Chemist's Society, IL (US); B.F.WARD et al, Seiten 219-224**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Höffkes, Horst, Dr.**
**Carlo Schmid-Str. 113**
**W-4000 Düsseldorf-Hellerhof(DE)**
Erfinder: **Seidel, Kurt**
**Nosthoffenstr. 59**
**W-4000 Düsseldorf 13(DE)**
Erfinder: **Giede, Karl**
**Schlehenweg 12**
**W-4010 Hilden(DE)**
Erfinder: **Struve, Alfred, Dr.**
**Am Eichelkamp 53**
**W-4010 Hilden(DE)**
Erfinder: **Gruber, Bert, Dr.**
**Marbacher Str. 7**
**W-4000 Düsseldorf 13(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Mittel zum Waschen oder Spülen der Haare in Form einer wäßrigen oder wäßrig-alkoholischen Zubereitung mit einem Gehalt an wasserlösliche ionischen Polymeren und wasserlöslichen Salzen von Di- oder Tricarbonsäuren.

Es ist bekannt, Haarwaschmitteln wasserlösliche ionische Polymere zuzusetzen, um gleichzeitig mit der Reinigung der Haare eine Verbesserung der haarkosmetischen Eigenschaften, insbesondere der Kämmbarkeit oder der Frisierbarkeit und des Haltes der Haare zu erreichen. Als geeignete ionische Polymere für diesen Zweck sind kationische, amphotere bzw. zwitterionische und auch anionische Polymere schon vorgeschlagen worden. Auch Mischungen von Kationpolymeren und Anionpolymeren, Mischungen aus kationischen und amphoteren Polymeren und Mischungen aus anionischen und amphoteren Polymeren sind schon für diesen Zweck vorgeschlagen worden.

Es ist auch bereits vorgeschlagen worden, Mitteln zum Spülen der Haare, vor allem nach der Haarwäsche mit üblichen Haarwaschmitteln ohne konditionierende Zusätze, wasserlösliche ionische Polymere zur Verbesserung der haarkosmetischen Eigenschaften zuzusetzen.

Ein Problem bei all diesen Produkten, die nach der Anwendung mit klarem Wasser aus dem Haar ausgespült werden, besteht darin, daß ein großer Teil der enthaltenen ionischen Polymeren und auch anderer haarkosmetischer Wirkstoffe dabei aus dem Haar ausgespült werden, so daß die kosmetischen Effekte nicht in ausreichendem Maße zur Geltung kommen. Es hat daher nicht an Vorschlägen gefehlt, die Abscheidung der genannten ionischen Polymeren auf dem Haar und damit die haarkosmetische Wirkung durch Zusätze zu den Haarwasch- und Haarspülmitteln zu verbessern. Aus EP-A-56 595 ist bekannt, daß sich die Kämmbarkeit der Haare durch Haarbehandlungsmittel stark verbessern läßt, die eine Kombination aus Betainen und aliphatischen organischen Säuren enthalten. Die EP-A-188 216, die nicht vorveröffentlicht ist, offenbart als Träger für Haarfärbemittel eine Mischung aus Seifen von Fettsäuren und wasserlöslichen kationischen Polymeren sowie aliphatischen oder alicyclischen Dicarbonsäuren und/oder speziellen alkoxylierten Aminen.

Weiterhin ist aus der japanischen Offenlegungsschrift 78/06438 (referiert bei Chemical Abstracts als Referat 88:158285y) bekannt, daß bestimmte Hydroxytricarbonsäuren wie die 3-Hydroxy-3,4-dicarboxypentadecansäure die Homogenität und die Absorbierbarkeit kosmetischer Produkte erhöhen.

Die bekannten technischen Lösungsversuche befriedigen jedoch noch nicht die hohen kosmetischen Erwartungen der Verbraucher an die konditionierenden und strukturverbessernden Eigenschaften von Haarwaschmitteln und Haarnachspülmitteln. Insbesondere ist keine brauchbare Möglichkeit bekannt, gleichzeitig mit der Abscheidung der Polymeren auch die Abscheidung anderer, in diesen Mitteln solubilisierter oder dispergierter wasserunlöslicher haarkosmetischer Wirkstoffe auf dem Haar zu erhöhen.

Es wurde gefunden, daß Mittel zum Waschen oder Spülen von Haaren in Form einer wäßrigen oder wäßrig-alkoholischen Zubereitung mit einem Gehalt an ionischen Polymeren eine merklich verbesserte haarkosmetische Wirkung aufweisen, wenn sie zusätzlich bestimmte Carbonsauren in Form eines wasserlöslichen Salzes enthalten.

Gegenstand der Erfindung ist daher ein Mittel zum Waschen oder Spülen der Haare in Form einer wäßrigen oder wäßrig-alkoholischen Zubereitung mit einem Gehalt an wasserlöslichen ionischen Polymeren, dadurch gekennzeichnet, daß 0,1 - 20 Gew.-% einer gesättigten oder ungesättigten aliphatischen oder alicyclischen oder aromatischen Di- oder Tricarbonsäure mit 6 - 44 C-Atomen, bei der zwei der Carboxylgruppen durch wenigstens 3 C-Atome voneinander getrennt sind, in Form eines wasserlöslichen Salzes enthalten enthalten sind, und das Mittel frei von Haarfarbstoff-Vorprodukten ist.

Als ionische Polymere für die erfindungsgemäßen Mittel eignen sich synthetische Polymere oder Derivate von natürlich vorkommenden Polymeren mit Molekulargewichten von 500 - 5000000, die so viele ionische Gruppen in der Polymerkette oder an diese gebunden enthalten, daß sie in Form von Salzen wasserlöslich sind. Wasserlöslich im Sinne der vorliegenden Erfindung heißt, daß wenigstens 0,1 Gew.-% des ionischen Polymeren in Salzform bei 20 °C in Wasser löslich sein sollte.

Als wasserlösliche kationische Polymere kommen praktisch alle Polymeren, bevorzugt im Molekulargewichtsbereich von 1000 bis 3 000 000, in Frage, die entweder in der Polymerkette freie oder alkylsubstituierte Aminogruppen oder quartäre Ammoniumgruppen enthalten oder an die Polymerkette direkt oder über Zwischenglieder gebundene, sekundäre oder tertiäre Aminogruppen oder quartäre Ammoniumgruppen tragen. Diese Aminogruppen oder quartären Ammoniumgruppen können auch Glieder von 5- oder 6-gliedrigen Ringsystemen sein, z.B. von Morpholin, Piperidin-, Piperazin- oder Indazol-Ringen. Zahlreiche Beispiele für solche wasserlöslichen kationischen Polymeren sind z.B. in US-PS 4.240.450 näher beschrieben. Darüber hinaus sind zahlreiche weitere wasserlösliche kationische Polymeren literaturbekannt. Bevorzugt geeignet sind wasserlösliche Homo- oder Mischpolymerisate mit Einheiten der allgemeinen Formel

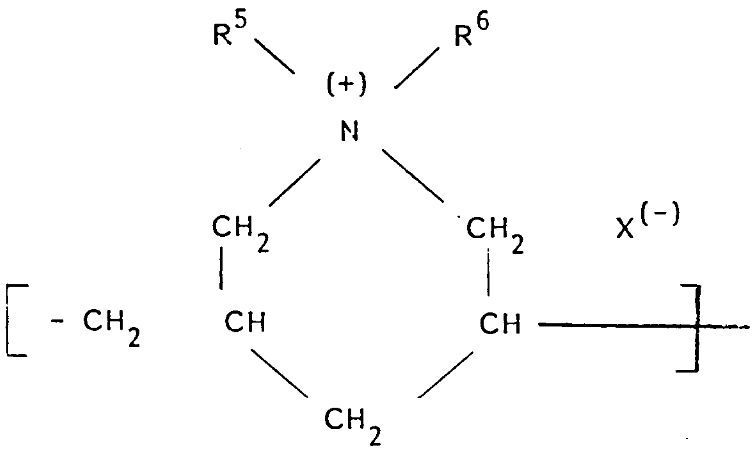

in der $R^5$ und $R^6$ Wasserstoff oder Alkylgruppen mit 1 - 4 C-Atomen oder Hydroxyalkylgruppen mit 2 - 4 C-Atomen sind und $X^{(-)}$ ein Chlorid-, Bromid-, Hydrogensulfat-, Methoxy- oder Ethoxysulfat-Anion ist. Die Herstellung solcher Polymeren ist aus US-PS 3.912.808 bekannt. Handelsprodukte dieser Struktur sind z.B. Merquat$^{(R)}$ 100 und Marquart$^{(R)}$ 550 (Quaternium 41).

Weitere bevorzugt geeignete kationische Polymeren sind z.B. Celluloseether, deren Anhydroglucoseeinheiten jeweils 1 -3 über Ethersauerstoff gebundene Substituenten mit quartären Ammoniumgruppen tragen. Solche Polymeren sind z.B. aus US-PS 3.472.840 bekannt. Ein Handelsprodukt mit dieser Struktur ist z.B. Polymer JR$^{(R)}$400.

Weitere besonders geeignete kationische Polymeren sind z.B. die aus der US-PS 3.910.862 bekannten und z.B. unter der Handelsbezeichnung Gafquat$^{(R)}$734 und 755 erhältlichen quartären Polyvinylpyrrolidon-Copolymerisate, die aus US-PS 3.632.559 bekannten und z.B. unter der Handelsbezeichnung Cartaretine$^{(R)}$F 4 erhältlichen Copolymeren aus Adipinsäure und Dimethylaminohydroxypropyl-diethylentriamin und die aus US-PS 4.157.388 bekannten und z.B. unter der Handelsbezeichnung Mirapol$^{(R)}$A15 erhältlichen quartären polymeren Harnstoffderivate.

Als wasserlösliche amphotere Polymere sind solche Polymeren und Copolymeren zu verstehen, deren Polymerkette sowohl Einheiten mit kationischen als auch Einheiten mit anionischen Gruppen enthält, wobei die kationischen Gruppen freie oder alkylsubstituierte Aminogruppen und die anionischen Gruppen, Carboxylatgruppen, Sulfonatgruppen oder Sulfatestergruppen sein können, oder deren Polymerkette Einheiten mit ampholytischen Gruppen enthält. Solche Polymeren werden z.B. durch Copolymerisation aus kationischen, anionischen und ggf. nichtionischen Monomeren oder durch Copolymerisation aus ampholytischen und nichtionischen Monomeren erhalten. Als zwitterionische Polymere werden solche verstanden, die analog dem amphoteren Polymeren aufgebaut sind, aber anstelle der Aminogruppen quartäre Ammoniumgruppen aufweisen. Geeignete amphotere und zwitterionische Polymere lassen sich z.B. durch Copolymerisation aus anionischen Monomeren wie Acrylsäure, Methacrylsäure und Maleinsäure und kationischen Monomeren, z.B. Dialkylaminoalkyl-methacrylat oder -acrylat, Dialkylaminoalkylmethacrylamid oder -acrylamid nach US-PS 3.836.537 herstellen. Ein geeignetes Handelsprodukt dieser Art, ein Octylacrylamid/Acrylat/Butylaminoethylmethacrylat-Copolymerisat, ist unter der Handelsbezeichnung Amphomer$^{(R)}$ erhältlich. Andere geeignete zwitterionische Polymere sind durch Copolymerisation zwitterionischer Monomerer, z.B. des N-methacryloxyethyl-N,N-dimethylamino-propionat, welches z.B. nach US-PS 2.744.130 zugänglich ist, mit nichtionischen Monomeren herstellbar. Weitere geeignete amphotere und zwitterionische Polymere sind in DE-OS 3.044.738 beschrieben.

Als wasserlösliche anionische Polymere eignen sich die Alkali-, Ammonium- und Alkanolammoniumsalze von Polymerisaten mit Carbonsäure- oder Sulfonsäuregruppen, welche direkt oder über Zwischenglieder an die Polymerkette gebunden sind, mit einem Molekulargewicht von 500 - 5 000 000. Solche Polymerisate lassen sich z.B. durch Homo- oder Copolymerisation von Acryl- oder Methacrylsäure, Maleinsäure oder Maleinsäureanhydrid, Fumarsäure, Itaconsäure, mit nichtionischen polymerisierbaren Monomeren wie z.B. Acryl- oder Methacrylsäureestern, Acryl- oder Methacrylamid, Vinylestern wie z.B. Vinylacetat, Vinylpropionat, Vinylchlorid, Vinylethern wie z.B. Vinylmethylether, Vinylbenzol, Allyl- und Methallylestern herstellen. Besonders geeignet sind die carboxylgruppenhaltigen Polymerisate, die durch Hydrolyse von Poly-(Ethylen/Maleinsäureanhydrid) und Poly-(Methylvinylether/Maleinsäureanhydrid) oder durch Hydrolyse von Polyacrylsäure- und Polymethacrylsäureestern erhalten werden. Solche aus Methyl-vinylether und Maleinsäureanhydrid erhältliche anionische Copolymere sind unter der Handelsbezeichnung Grantrez$^{(R)}$AN-Polymere erhältlich.

Andere besonders gut geeignete anionische wasserlösliche Polymere sind Polyaldehydocarbonsäuren mit einem mittleren molekulargewicht von 600 - 10 000, die durch oxidative Homopolymerisation von Acrolein oder durch oxidative Copolymerisation von Acrolein und Acrylsäure hergestellt werden. Die Salze dieser Polyaldehydocarbonsäuren können durch Disproportionierung (nach Cannizzaro) in Gegenwart von Laugen in die Salze der Polyhydroxycarbonsäuren überführt werden. Diese Cannizzaro-Reaktion kann in Gegenwart von Formaldehyd unter gleichzeitiger Aldol-Reaktion durchgeführt werden, wobei Polyhydroxycarbonsäure-Salze gebildet werden, die in alpha-Stellung zu den aus der Aldehydgruppe gebildeten Carboxylat- oder Hydroxymethylgruppen eine weitere Hydroxymethylgruppe tragen. Die Herstellung dieser Polymeren ist z.B. in DE-PS 1.904.940 beschrieben. Solche Poly-(aldehydocarbonsäuren) und Poly-(hydroxycarbonsäuren) und deren Natriumsalze sind im Handel unter der Bezeichnung POC (Degussa) erhältlich.

Die erfindungsgemäßen Mittel zum Waschen und Spülen der Haare enthalten bevorzugt wasserlösliche ionische Polymere der vorgenannten kationischen, amphoteren, zwitterionischen oder anionischen Struktur in einer Menge von 0,1 - 5,0 Gew.-%.

Als aliphatische, alicyclische oder aromatische Dicarbonsäuren mit 6 - 44 C-Atomen eignen sich z.B. Adipinsäure, Azelainsäure, Heptadecan-1,8-dicarbonsäure, Heptadecan-1,9-dicarbonsäure, Heptadecan-1,17-dicarbonsäure, Terephthalsäure, alkylsubstituierte Terephthalsäuren mit bis zu 30 C-Atomen in den Alkylgruppen und Gemische dieser Dicarbonsäuren. Weiterhin eignen sich die die Addukte von ungesättigten Fettsäuren wie Undecylensäure, Ölsäure, Palmitoleinsäure, Linolsäure, Linolensäure, konjugierten mehrfach ungesättigten Fettsäuren oder Erucasäure an einfach ungesättigte Carbonsäuren wie Acryl- oder Methacrylsäure, Maleinsäure bzw. Maleinsäureanhydrid oder Fumarsäure und die durch Hydrierung der noch vorhandenen Doppelbindungen entstehenden gesättigten Säuren. Unter diesen En-Addukten kommt dem Addukt aus Linolsäure und Acrylsäure, welches nach dem in US-PS 3.753.968 beschriebenen Verfahren hergestellt werden kann, besondere Bedeutung zu. Das dort aufgeführte Diels-Alder-Addukt, eine $C_{21}$-Dicarbonsäure, besteht aus einem Gemisch aus 6-Carboxy-4-hexyl-2-cyclohexen-1-octansäure und 5-Carboxy-4- hexyl-2-cyclohexen-1-octansäure und ist in J. Am. Oil Chem. Soc. 52, (1975), S. 219 - 224 näher beschrieben und unter der Handelsbezeichnung Westvaco Diacid(R)1550 erhältlich.

Die durch Verseifung von En-Addukten aus Undecylensäuremethylester und Maleinsäureanhydrid erhältliche Tricarbonsäure, eine 4-Dodecen-1,2,4-tricarbonsäure, ist z.B. in J. Am. Chem. Soc. 68, (1946), S. 1373-1376 beschrieben. Sie läßt sich durch Hydrierung der Doppelbindung in die gesättigte 1,2,4-Dodecantricarbonsäure überführen.

Weitere En-Addukte,die durch Addition von Maleinsäure, Maleinsäureanhydrid, Maleinsäureester oder Fumarsäure an ungesättigte Fettsäuren und Fettsäureester, z.B. an Ölsäure oder Linolsäure, und ggf. Verseifung der Addukte erhältlichen Tricarbonsäuren sind in US-PS 2.188.882 beschrieben. Geeignete Dicarbonsäuren sind auch die durch thermische En-Addition aus ungesättigten Fettsäuren, z.B. aus Ölsäure und Linolsäure herstellbaren ungesättigten Dimerfettsäuren, die z.B. in J. Am. Oil Chem. Soc. 39, (1962), S. 534-545 näher beschrieben sind und die daraus durch Hydrierung der Doppelbindungen erhältlichen gesättigten Dicarbonsäuren. Solche sog. Dimerfettsäuren sind z.B. unter der Bezeichnung Empol(R)1010 (Unilever Emery) im Handel erhältlich.

Eine gewisse Wirksamkeit im Sinne der Erfindung, die jodoch deutlich unter der der beanspruchten Säuren liegt, weisen auch die substituierten Bernsteinsäuren auf, z.B. die 2-Alkyl- und 2-Alkenylbernsteinsäuren. Die Alkenylbernsteinsäuren sind z.B. nach dem Verfahren gemäß US-PS 2.411.215 aus Maleinsäureanhydrid und Monoolefinen erhältlich. Die entsprechenden gesättigten Alkylbernsteinsäuren können aus diesen durch Hydrierung der Doppelbindung gewonnen werden.

In den erfindungsgemäßen Mitteln zum Waschen oder Spülen der Haare sind die genannten Di- und Tricarbonsäuren in Form ihrer wasserlöslichen Salze, bevorzugt der Natrium, Kalium, Ammonium Mono-, Di- oder Trialkanolammoniumsalze mit 2 - 4 C-Atomen in der Alkanolgruppe enthalten. Es sollte wenigstens 0,1 Gew.-% der Di- oder Tricarbonsäure in Form dieser Salze in der Zubereitung löslich sein. Bevorzugt geeignet sind die 5(6)-Carboxy-4-hexyl-2-cyclohexen-1-octansäure (Westvaco Diacid 1550) und die isomeren Carboxystearinsäuren, insbesondere das Gemisch aus Heptadecan-1,8-dicarbonsäure und Heptadecan-1,9-dicarbonsäure. Die Salze dieser Säuren besitzen über den Effekt der erhöhten Polymerdeposition auf dem Haar hinaus ein besonders hohes Solubilisationsvermögen für wasserunlösliche Komponenten, insbesondere für kosmetische Öl- und Fettkomponenten.

Besonders wirksame, erfindungsgemäße Mittel zum Waschen oder Spülen der Haare werden erhalten, wenn zusätzlich zu den ionischen Polymeren und der Dicarbonsäure noch eine quartäre Ammoniumverbindung, bevorzugt eine der allgemeinen Formel

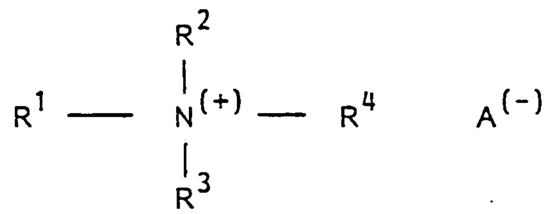

in der $R^1$ eine Alkyl-, Alkenyl- oder Hydroxyalkylgruppe mit 8 - 22 C-Atomen oder eine Gruppe $R^5$-CONH-$(CH_2)_x$-, in der $R^5$ eine Alkylgruppe mit 7 - 21 C-Atomen und x eine ganze Zahl von 2 - 4 ist, $R^2$ und $R^3$ eine Alkylgruppe mit 1 - 4 C-Atomen, eine Gruppe - $(Ca_nH_{2n}O)y$-H, in der n = 2 oder 3 und y eine Zahl von 1 - 50 ist und $R^4$ eine Benzylgruppe ist oder eine der für $R^1$, $R^2$ oder $R^3$ genannten Bedeutungen hat, und $A^{(-)}$ ein Chlorid-, Bromid-, Methoxysulfat-, Ethoxysulfat-, Hydrogensulfatoder Hydrogenphosphat-Anion darstellt, in einer Menge von 0,1 - 5,0 Gew.-% enthalten ist. Beispiele für quartäre Ammoniumverbindungen, die in die erfindungsgemäßen Mittel eingesetzt werden können, sind:
Cetyltrimethylammonium-chlorid,
Stearyldimethylbenzylammoniumchlorid,
Distearyldimethylammoniumchlorid,
Acylamidopropyl-dimethyl-ethylammonium-ethoxysulfat
(Ein Produkt dieses letzteren Typs, wobei die Acyl-Gruppe von der Lanolinfettsäure abgeleitet ist, ist unter der Bezeichnung Lanoquat$^{(R)}$ 1756 (Quaternium 33 and Ethyl Hexanol) im Handel.)

Acylamidopropyl-trimethylammonium-methoxysulfat

(Ein Produkt dieser Struktur, wobei die Acylgruppe von der Ricinolfettsäure abgeleitet ist, ist unter der Bezeichnung Rewoquat$^{(R)}$ RTM50 im Handel.) Viele weitere geeignete quartäre Ammoniumverbindungen sind z.B. in Hugo Janistyn "Handbuch der Kosmetika und Riechstoffe", Band 111, 2. Auflage (1973), Dr.A. Hüthig Verlag GmbH, Heidelberg auf Seite 419 - 420 aufgeführt.

Durch den Zusatz der quartären Ammoniumverbindung wird der haarkonditionierende Effekt der erfindungsgemäßen Mittel noch erheblich gesteigert. Der Zusatz ist besonders für erfindungsgemäße Mittel zu empfehlen, die zur Verwendung als Haarnachspülmittel bestimmt sind.

Wie weiter oben ausgeführt, sind die erfindungsgemäßen Mittel aufgrund der darin enthaltenen Kombination aus ionischen Polymeren und Dicarbonsäuren nicht nur dadurch wirksam, daß sie einen verstärkten Effekt des haarkosmetisch wirksamen Polymeren auf der Haarfaser bewirken sondern darüber hinaus wird die Abscheidung von in diesen Mitteln solubilisierten oder dispergierten wasserunlöslichen Komponenten auf der Haarfaser begünstigt. Solche wasserunlöslichen haarkosmetischen Wirkstoffe können z.B. sein Antischuppenwirkstoffe, wie die Zinksalze des 1-Hydroxy-2-pyridinthions oder Filtersubstanzen für Ultraviolett-Strahlung wie z.B. der N.N-Dimethyl-4-aminobenzoesäure-2-ethylhexylester (Escalol$^{(R)}$507) oder kosmetische Öl- und Fettkomponenten, z.B. Fettalkohole wie Cetylalkohol und Stearylalkohol, Fettsäurepartialglyceride wie Stearinsäure-mono- und diglycerid, Fettsäure-Fettalkoholester wie z.B. Decyloleat, flüssige verzweigte Alkohole wie z.B. 2-Octyl-dodecanol oder 2-Hexyldecanol, Fettsäuretriglyceride wie z.B. Capryl-Caprinsäure-triglycerid, Fettsäureester wie z.B. Isopropylmyristat, 2-Ethylhexylstearat, n-Butylstearat und andere übliche kosmetische Öle und Fettkomponenten. Erfindungsgemäße Mittel zur Verwendung als Haarnachspülmittel enthalten bevorzugt 0,1 - 10 Gew.-% einer wasserunlöslichen kosmetischen Öl- oder Fettkomponente.

In den erfindungsgemäßen Mitteln zum Waschen oder Spülen der Haare wird die Einarbeitung vieler wasserunlöslicher Wirkstoffe, Öl- und Fettkomponenten durch die solubilisierende Wirkung der Dicarbonsäure-Salze erheblich erleichtert.

Wenn man erfindungsgemäße Haarnachspülmittel mit einem Gehalt an wasserunlöslichen Öl- oder Fettkomponenten klar solubilisiert formulieren will, empfiehlt es sich, die Dicarbonsäure in einem Überschuß von etwa 2 : 1 bis 50 : 1 zum Gewicht der Öl- oder Fettkomponente anzuwenden. Wenn im Einzelfall eine klare Solubilisierung auf diese Weise nicht erreicht wird, oder wenn ein geringerer Anteil an Dicarbonsäure erwünscht ist, kann die Solubilisierung durch Zusatz eines niedermolekularen Alkohols, z.B. von Ethanol oder Isopropanol erreicht werden. Mengen von bis zu 40 Gew.-% des niedermolekularen Alkohols sind meist ausreichend. Die zur Solubilisierung erforderliche Menge an Alkohol ist in den erfindungsgemäßen Mittel stets niedriger als in in Mitteln, die keine Dicarbonsäure-Salze enthalten.

Erfindungsgemäße Mittel zur Verwendung als Haarwaschmittel enthalten zusätzlich waschwirksame oberflächenaktive Mittel, bevorzugt 1 - 25 Gew.-% anionischer, ampholytischer, zwitterionischer oder

nichtionogener Tenside.

Als anionische Tenside kommen vor allem synthetische Sulfat- oder Sulfonattenside in Frage. Als Produkte dieses Typs eignen sich z.B. die Alkali-, Ammonium- oder Alkanolammoniumsalze mit 2 - 4 C-Atomen in der Alkanolgruppe, von Alkyl($C_{10}$-$C_{18}$)-sulfaten, Alkyl-(poly)-glykol-ethersulfaten mit 10 - 16 C-Atomen in der Alkylgruppe und 1 - 12 Glykolethergruppen, Alkansulfonate mit 10 - 18 C-Atomen, Alken- und Hydroxyalkansulfonate, wie sie bei der Sulfonierung von alpha-Olefinen mit 10 - 18 C-Atomen erhalten werden, Fettsäurealkylolamid- und Fettsäurealkylolamidpolyglykolether-sulfate, Fettsäuremonoglyceridsulfate, Sulfobernsteinsäuremonoalkylester, Acyltauride und Acylisethionate mit 10 - 18 C-Atomen in der Acylgruppe. Beispiele für ampholytische Tenside sind N-($C_8$-$C_{18}$)-Alkyl-beta-aminopropionsäuren oder N-Hydroxyethyl-N-kokosacylamidopropyl-glycin, Beispiele für zwitterionische Tenside (Betaintenside) sind N-Kokosalkyl-N.N-dimethylglycin oder N-Kokosacylamidopropyl-N.N-dimethylglycin.

Bevorzugt sind in erfindungsgemäßen Mitteln zum Waschen der Haare nichtionogene Tenside enthalten. Geeignet sind z.B. Anlagerungsprodukte von 6 - 20 Mol Ethylenoxid an Fettalkohole mit 12 - 18 C-Atomen, an Fettsäuren mit 12 - 18 C-Atomen, an Alkylphenole mit 8 - 12 C-Atomen in der Alkylgruppe, an Fettsäurealkylolamide, an Fettsäuremono- und Diglyceride, an Sorbitanfettsäureester. Weitere geeignete nichtionogene Tenside sind Fettsäuremono- und diethanolamide und Aminoxid-Tenside. Geeignete Aminoxid-Tenside sind z.B. das Kokosalkyl-dimethylaminoxid oder das Kokosacylamidopropyl-dimethylaminoxid. Weitere besonders geeignete nichtionogene Tenside sind die Alkylglucoside, wie sie z.B. gemäß US-PS 3.598.865 aus Monosacchariden und einwertigen Alkoholen mit 8 - 25 C-Atomen hergestellt werden können.

Neben den obligatorischen Komponenten - wasserlöslichen ionischen Polymeren und Di- oder Tricarbonsäuren - und den gegebenenfalls vorhandenen quartären Ammoniumverbindungen, wasserunlöslichen kosmetischen Öl- oder Fettkomponenten und waschaktiven oberflächenaktiven Mitteln können die erfindungsgemäßen Mittel zum Waschen und Spülen der Haare übliche Zusatz- und Stellmittel wie z.B. Verdickungsmittel, etwa vom Typ der nichtionogenen wasserlöslichen Polymeren wie Polyvinylpyrrolidon, Hydroxyethylcellulose oder Polyethylenglykole, Trübungs- und Perlglanzmittel z.B. Ethylenglykoldistearat oder Triethylenglykoldistearat, pH-Wert-Stabilisatoren (Puffer) wie z.B. Alkali- oder Ammoniumphosphate oder Citrate, Konservierungsmittel wie z.B. Formaldehyd, p-Hydroxybenzoesäureester oder 2-Methyl-4-isothiazolin-3-one, haarkosmetische Wirkstoffe wie z.B. Antischuppenwirkstoffe, Sebostatika, Vitamine, Pflanzenextrakte, Proteinderivate sowie Farb- und Duftstoffe in den in solchen Mitteln üblichen Mengen enthalten.

Die folgenden Beispiele sollen den Gegenstand der Erfindung näher erläutern:

**Beispiele**

1. Haarnachspülmittel

| | 1.1 | 1.2 |
|---|---|---|
| Cetyltrimethylammoniumchlorid | - | 1 Gew.% |
| Stearyldimethylbenzylammonium-chlorid | 1,0 Gew.% | - |
| Gafquat(R)755 | 4,0 Gew.% | 4 Gew.% |
| Sonnenblumenkernöl | 0,5 Gew.% | - |
| Myristylalkohol | - | 1 Gew.% |
| Parfümöl | - | 0,2 Gew.% |
| Westvaco Diacid(R)1550 | 1 | 10 Gew.% |
| Triethanolamin | bis pH=7,5 | bis pH=7,7 |
| Wasser | ad 100 Gew.% | ad 100 Gew.% |
| Aussehen bei 20°C | Emulsion | klar |

2. Nachweis der erhöhten Abscheidung von solubilisierten Wirkstoffen auf dem Haar

Prüfrezepturen (Haarnachspülmittel)

| | Erfindung | | Vergleich |
|---|---|---|---|
| | 2.1 | 2.2 | 2.3 |
| Polymer JR(R)400 | 1 Gew.% | 1 Gew.% | - |
| Westvaco Diacid(R)1550 | 10 Gew.% | 10 Gew.% | 10 Gew.% |
| Lanoquat(R)DES 50 | 5 Gew.% | - | 5 Gew.% |
| Escalol(R)507 | 5 Gew.% | 5 Gew.% | 5 Gew.% |
| Isopropanol | 25 Gew.% | 35 Gew.% | 25 Gew.% |
| Triethanolamin | bis pH=7,5 | b.pH=7,5 | b.pH=7,5 |
| Wasser | ad 100 Gew.% | ad 100Gew.% | ad 100Gew.% |
| Adsorbierte Menge Escalol 507 (mg/g Haar) | 3,65 | 1,13 | 0,39 |

Standardisiertes Humanhaar, welches durch einmaliges Blondieren und einmaliges Dauerwellen vorbehandelt worden war, wurde mit einer definierten Menge der Haarnachspülmittel 2.1, 2.2 und 2.3 behandelt und mit Wasser gründlich ausgespült. Das Spülwasser wurde mit dem Haarnachspülmittel wieder vereinigt und die Menge des Escalol 507 nach der Haarbehandlung ultraviolett-spektrometisch bestimmt. Aus der Differenz zur eingesetzten Menge Escalol 507 und durch Bezug auf die Gewichtsmenge des eingesetzten Haars wurde die an das Haar adsorbierte Menge Escalol 507 (mg/g Haar) errechnet.

3. Shampoo

| | |
|---|---|
| Triton(R)CG 110 | 5 Gew.-% |
| Aminoxid WS(R)35 | 3 Gew.-% |
| Rewoquat(R)RTM 50 | 1,2 Gew.-% |
| POC HS 5060(R) | 5 Gew.-% |
| Westvaco Diacid 1550 | 7,5 Gew.-% |
| Triethanolamin | bis pH = 7,5 |
| Parfümöl | 0,2 Gew.-% |
| Wasser | ad 100 Gew.-% |

In den Rezepturbeispielen wurden die folgenden Handelsprodukte verwendet:

Lanoquat DES 50: N-Lanolinfettsäureamidopropyl-N,N-dimethyl-N-ethyl-ammonium-ethosulfat, 50 %ig in Ethylhexandiol (Emery Industries)

Westvaco Diacid 1550: Gemisch aus 6-Carboxy-4-hexyl-2-cyclohexen-1-octansäure mit 5-Carboxy-4-hexyl-2-cyclohexen-1-octansäure (Firma Westvaco)

Polymer JR 400: Kationisches Celluloseetherderivat mit quartären Ammoniumgruppen (Union Carbide)

Gafquat(R)755: Quartäres Polyvinylpyrrolidon-Copolymerisat (20 Gew.-%ig in Wasser) GAF-Corp., New York

Escalol(R)507: 4-Dimethylaminobenzoesäure-2-ethylhexylester, UV-Filtersubstanz (van Dyk)

Triton(R)CG 110: Alkylglucosid der allgemeinen Formel

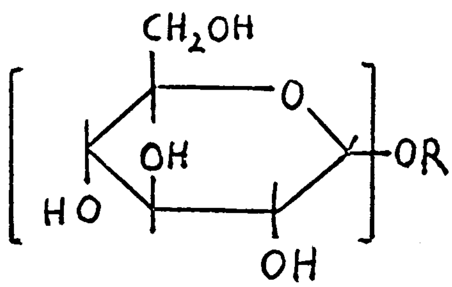

X = 1 - 5

R = $C_8/C_{10}$- Alkylgruppe

(Rohm & Haas)

Rewoquat RTM 50: Ricinoleylamidopropyl-trimethylammoniummethoxysulfat, 40 Gew.-% in Wasser (REWO)

Aminoxid WS 35: Kokosfettacyl ($C_{12}$-$C_{18}$)-amido-propyldimethylamin-oxid 35 Gew.-% in Wasser (TH. Goldschmidt)

POC HS 5060: Poly-(aldehydocarbonsäure), mittleres Molekulargewicht ca. 4000, 40 Gew.-% in Wasser (DEGUSSA)

**Patentansprüche**

**1.** Mittel zum Waschen oder Spülen der Haare in Form einer wäßrigen oder wäßrig-alkoholischen Zubereitung mit einem Gehalt an wasserlöslichen ionischen Polymeren, dadurch gekennzeichnet, daß 0,1 - 20 Gew.-% einer gesättigten oder ungesättigten aliphatischen oder alicyclischen oder aromatischen Di- oder Tricarbonsäure mit 6 - 44 C-Atomen, bei der zwei der Carboxylgruppen durch wenigstens 3 C-Atome voneinander getrennt sind, in Form eines wasserlöslichen Salzes enthalten enthalten sind, und das Mittel frei von Haarfarbstoff-Vorprodukten ist.

**2.** Mittel nach Anspruch 1, dadurch gekennzeichnet, daß als Dicarbonsäure die 5(6)-Carboxy-4-hexyl-2-cyclohexen-1-octansäure in Form des Natrium-, Kalium-, Ammonium-, Mono-, Di- oder Triethanolammoniumsalzes enthalten ist.

**3.** Mittel nach Anspruch 1 oder 2, dadurch gekennzeichnet, daß als wasserlösliches ionisches Polymeres ein kationisches, ampholytisches, zwitterionisches oder anionisches Polymeres in einer Menge von 0,1 - 5,0 Gew.-% enthalten ist.

**4.** Mittel nach Anspruch 1 - 3, dadurch gekennzeichnet, daß zusätzlich eine quartäre Ammoniumverbindung der allgemeinen Formel

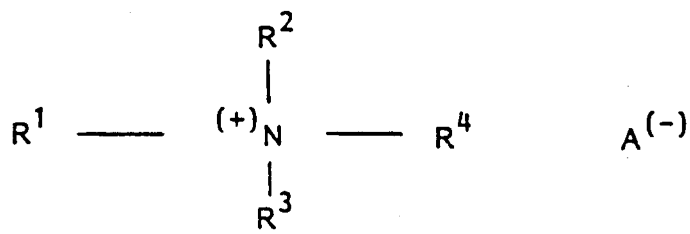

in der $R^1$ eine Alkyl-, Alkenyl- oder Hydroxyalkylgruppe mit 8 - 22 C-Atomen oder eine Gruppe $R^5$-CONH-$(CH_2)_x$-, in der $R^5$ eine Alkylgruppe mit 7 - 21 C-Atomen und x eine ganze Zahl von 2 - 4 ist, $R^2$ und $R^3$ eine Alkylgruppe mit 1 - 4 C-Atomen, eine Gruppe $-(C_nH_{2n}O)_yH$, in der n = 2 oder 3 und y eine Zahl von 1 - 50 ist und $R^4$ eine Benzylgruppe ist oder eine der für $R^1$, $R^2$ und $R^3$ genannten Bedeutungen hat, und $A^{(-)}$ ein Chlorid-, Bromid-, Methoxysulfat-, Ethoxysulfat-, Hydrogensulfat- oder Hydrogenphosphat-Anion darstellt, in einer Menge von 0,1 - 5,0 Gew.-% enthalten ist.

**5.** Mittel nach Anspruch 1 - 4 zur Verwendung als Haarnachspülmittel, dadurch gekennzeichnet, daß zusätzlich 0,1 - 10 Gew.-% einer wasserunlöslichen kosmetischen Öl- oder Fettkomponente enthalten ist.

**6.** Mittel nach Anspruch 5, dadurch gekennzeichnet, daß die Di- oder Tricarbonsäure zur wasserunlöslichen Öl- oder Fettkomponente im Gewichtsverhältnis 2 : 1 bis 50 : 1 enthalten ist.

**7.** Mittel nach Anspruch 1 - 4 zur Verwendung als Haarwaschmittel, dadurch gekennzeichnet, daß zusätzlich 1 - 25 Gew.-% anionischer, ampholytischer, zwitterionischer oder nichtionogener Tenside enthalten sind.

**Claims**

**1.** Preparations for washing or rinsing hair in the form of an aqueous or aqueous-alcoholic formulation containing water-soluble ionic polymers, characterized in that they contain from 0.1 to 20% by weight of a saturated or unsaturated aliphatic or alicyclic or aromatic di- or tricarboxylic acid containing from 6 to 44 carbon atoms, in which two of the carboxyl groups are separated from one another by at least 3 carbon atoms, in the form of a water-soluble salt and in that the preparations are free from hair dye precursors.

**2.** Preparations as claimed in claim 1, characterized in that 5(6)-carboxy-4-hexyl-2-cyclohexene-1-octanoic acid in the form of the sodium, potassium, ammonium, mono-, di- or triethanolammonium salt is present as the dicarboxylic acid.

**3.** Preparations as claimed in claim 1 or 2, characterized in that a cationic, ampholytic, zwitterionic or anionic polymer is present as the water-soluble ionic polymer in a quantity of from 0.1 to 5.0% by weight.

**4.** Preparations as claimed in claims 1 to 3, characterized in that a quaternary ammonium compound corresponding to the following general formula

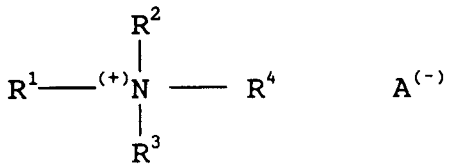

in which
$R^1$ is an alkyl, alkenyl or hydroxyalkyl group containing from 8 to 22 carbon atoms or a group $R^5$-CONH-$(CH_2)_x$-, where $R^5$ is an alkyl group containing from 7 to 21 carbon atoms and x is an integer of from 2 to 4, $R^2$ and $R^3$ represent a $C_{1-4}$ alkyl group, a group $-(C_nH_{2n}O)_y-H$, in which n = 2 or 3 and y is a number of from 1 to 50, $R^4$ is a benzyl group or has one of the meanings defined for $R^1$, $R^2$ and $R^3$ and $A^{(-)}$ is a chloride, bromide, methoxysulfate, ethoxysulfate, hydrogen sulfate or hydrogen phosphate anion, is additionally present in a quantity of from 0.1 to 5.0% by weight.

**5.** Preparations as claimed in claims 1 to 4 for use as hair rinses, characterized in that a water-insoluble cosmetic oil or fat component is additionally present in a quantity of from 0.1 to 10% by weight.

**6.** Preparations as claimed in claim 5, characterized in that the dicarboxylic acid or tricarboxylic acid is present in a ratio by weight to the water-insoluble oil or fat component of from 2:1 to 50:1.

**7.** Preparations as claimed in claims 1 to 4 for use as shampoos, characterized in that anionic, ampholytic, zwitterionic or nonionic surfactants are additionally present in a quantity of from 1 to 25% by weight.

**Revendications**

**1.** Produit pour le lavage ou le rinçage de la chevelure, sous la forme d'une préparation aqueuse ou aqueuse-alcoolique renfermant des polymères ioniques solubles dans l'eau, caractérisé en ce que 0,1 à 20 % en poids d'un acide di- ou tricarboxylique saturé ou insaturé, aliphatique, alicyclique ou aromatique, comportant 6 à 44 atomes de C, dans lequel deux des groupes carboxyle sont séparés par au moins 3 atomes de C, sont contenus sous la forme d'un sel soluble dans l'eau, et en ce que le produit est exempt de précurseurs de teintures capillaires.

**2.** Produit selon la revendication 1, caractérisé en ce qu'il contient comme acide dicarboxylique l'acide 5-(6)-carboxy-4-hexyl-2-cyclohexène-1-octanoïque, sous la forme d'un sel de sodium, de potassium, d'ammonium, de mono-, di- ou triéthanolammonium.

**3.** Produit selon la revendication 1 ou 2, caractérisé en ce qu'il contient comme polymère ionique soluble dans l'eau, un polymère cationique, ampholyte, zwitterionique ou anionique, dans une proportion de 0,1 à 5,0 % en poids.

**4.** Produit selon les revendications 1 à 3, caractérisé en ce qu'il contient en outre, en proportion allant de 0,1 à 5,0 % en poids, un composé d'ammonium quaternaire de formule générale

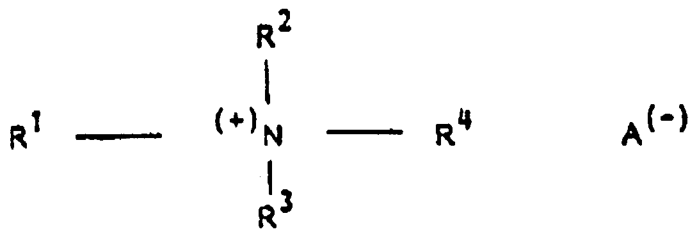

dans laquelle $R^1$ représente un groupe alkyle, alcényle ou hydroxyalkyle comportant 8 à 22 atomes de C ou un groupe $R^5$-CONH-$(CH_2)_x$-, dans lequel $R^5$ correspond à un groupe alkyle comprenant 7 à 21

atomes de C et x est un nombre entier de 2 à 4, $R^2$ et $R^3$ représentent chacun un groupe alkyle comportant 1 à 4 atomes de C, un groupe $-(C_nH_{2n}O)y-H$, dans lequel n = 2 ou 3 et y correspond à un nombre de 1 à 50, $R^4$ représente un groupe benzyle ou possède une des significations mentionnées pour $R^1$, $R^2$ ou $R^3$, et $A^{(-)}$ est un anion chlorure, bromure, méthoxysulfate, éthoxysulfate, hydrogènosulfate ou hydrogènophosphate.

**5.** Produit selon les revendications 1 à 4 pour utilisation comme agent de rinçage final de la chevelure, caractérisé on ce qu'il contient de surcroft 0,1 à 10 % en poids d'un composant cosmétique huileux ou gras insoluble dans l'eau.

**6.** Produit selon la revendication 5, caractérisé en ce que le rapport pondéral entre l'acide di- ou tricarboxylique et le composent huileux ou gras est compris dans l'intervalle de 2:1 à 50:1.

**7.** Produit selon les revendications 1 à 4, pour utilisation comme produit pour le lavage de la chevelure, caractérisé en ce qu'il contient en outre 1 à 25 % en poids de tensioactifs anioniques, ampholytes, zwitterioniques ou non ioniques.